# EUROPEAN PATENT APPLICATION

(11) **EP 2 921 164 A1**
(43) Date of publication of application: **23.09.2015**
(21) Application number: 13854692.4
(22) Date of filing: 16.10.2013
(51) Int. Cl.: A61K 8/72, A61K 8/37, A61K 8/02, A61Q 19/00

(54) **STICKY HYDROGEL COSMETIC COMPOSITION**

(30) Priority: 13.11.2012 KR 20120128313
(71) Applicant: Genic Co., Ltd., Seocho-gu, Seoul 137-889 (KR)
(72) Inventor: YOO, Hyun-Oh, Seoul 137-930 (KR); KIM, Jong-Chul, Seoul 120-788 (KR); YANG, Jin-A, Suwon-si Gyeonggi-do 441-848 (KR); CHOI, Eun-Kyoung, Hwaseong-si Gyeonggi-do 445-787 (KR); KIM, Min-Seok, Incheon 403-784 (KR)
(74) Representative: Vidon Brevets & Stratégie
(86) International application number: PCT/KR2013/009265
(87) International publication number: WO 2014/077519

(57) **Abstract**

The present invention relates to a sticky hydrogel cosmetic composition. According to the present invention, the sticky hydrogel cosmetic composition containing 0.1 to 1 wt% of sodium polyacrylate having an Na substitution degree of at least 50% as a hydrophilic polymer forms hydrogel containing a sufficient amount of a sticky mucilaginous substance while having excellent stability in formulation. Therefore, the present invention has excellent adhesiveness when applied to the skin, thereby allowing the absorption of moisture and ingredients into the skin to be maximized. Thus, the present invention can be widely used for the development of various cosmetics such as a mask pack.

## Description

### [Technical Field]

The present disclosure relates to a sticky hydrogel cosmetic composition, and more particularly, to a sticky hydrogel cosmetic composition containing 0.1% by weight to 1% by weight of sodium polyacrylate having an Na substitution degree of 50% or higher as a hydrophilic polymer, based on total weight, to form a hydrogel having a high content of sticky mucilage and high formulation stability so that the sticky hydrogel cosmetic composition may have high adhesion to skin and also allow skin to maximally absorb water and nutrients when the sticky hydrogel cosmetic composition is applied to skin.

### [Background Art]

Since hydrogel, as a material having a three-dimensional hydrophilic polymer reticular structure including purified water as a dispersion medium, contains a large amount of water and exhibits flexibility like natural tissue, hydrogel has been actively researched and developed in various pharmaceutical, beauty, and environmental industry fields, for applications including wound dressings, contact lenses, drugs, cosmetics, implants, wastewater treatment agents, etc. Recently, research has been conducted into imparting intelligent controlled-release characteristics to hydrogel for controlling delivery of components of the hydrogel, or imparting response characteristics, such as expansion or shrinkage, to hydrogel, in response to stimuli such as pH levels, temperature, electric fields, or light.

Particularly, hydrogel, which has a structure similar to that of human cell substrates, is inert, highly elastic, and highly permeable to oxygen and nutrients, thereby exhibiting superior biocompatibility, and thus receiving attention in the pharmaceutical and beauty industries. In the beauty industry, cosmetic compositions having an improved effect of removing keratin or moisturizing and supplying various nutrients by controlling the types and contents of components and using the elasticity, skin adhesion, and smooth tactility of hydrogel have been constantly developed (please refer to Korean Patent Application Publication No. 2012-0059199, Korean Patent No. 0653002, United States Patent Application Publication No. 2012/0121521, and others).

The inventors have researched methods of adjusting the appearance, texture, physical properties, and tactile sensation of hydrogel while maintaining the excellent properties of the hydrogel so as to provide cosmetic compositions giving a good sense of use and having good appearance and moisturizing ability, like the mucilage of snails having good water-containing ability, in addition to having high formulation stability. As a result of the research, the inventors have found that a sticky hydrogel cosmetic composition containing 0.1% by weight to 1% by weight of sodium polyacrylate having an Na substitution degree of 50% or higher as an aqueous polymer, based on the total weight forms a hydrogel having high formulation stability and a high content of sticky mucilage. Based on this knowledge, the inventors have invented the present invention.

### [Patent documents]

Patent document 1: Korean Patent Application Publication No. 2012-0059199
Patent document 2: Korean Patent No. 0653002
Patent document 3: United States Patent Application Publication No. 2012/0121521

### [Disclosure]

### [Technical Problem]

An aspect of the present disclosure may provide a hydrogel cosmetic composition having a high content of sticky mucilage and high formulation stability.

### [Technical Solution]

According to an aspect of the present disclosure, a sticky hydrogel cosmetic composition may include 0.1% by weight to 1% by weight of sodium polyacrylate having an Na substitution degree of 50% or higher as a hydrophilic polymer based on total weight.

According to an embodiment of the present disclosure, the sticky hydrogel cosmetic composition may additionally include 1% by weight to 5% by weight of a gelling polymer, 0.1% by weight to 10% by weight of a skin effective component, 10% by weight to 35% by weight of a polyvalent alcohol and 0.01% by weight to 0.05% by weight of a gelling rate control agent based on total weight, in addition to the hydrophilic polymer.

According to an embodiment of the present disclosure, the sticky hydrogel cosmetic composition may additionally include 0.1% by weight to 10% by weight of an oil, 0.1% by weight to 10% by weight of a surfactant, or both thereof, based on total weight as necessary.

According to an embodiment of the present disclosure, the sticky hydrogel cosmetic composition may additionally include other additives such as a preservation agent, a fragrance, a pH control agent and a thickener as necessary.

### [Advantageous Effects]

Since a sticky hydrogel cosmetic composition containing 0.1% by weight to 1% by weight of sodium polyacrylate having an Na substitution degree of 50% or higher as a hydrophilic polymer based on total weight according to embodiments of the present disclosure can maximize adsorption of water and components into skin by forming a hydrogel which has high formulation stability and is rich in sticky mucilage, thereby obtaining good adhesion properties when applying the hydrogel to skin, the sticky hydrogel cosmetic composition may be widely utilized in the development of various cosmetics such as mask packs, etc.

### [Description of Drawings]

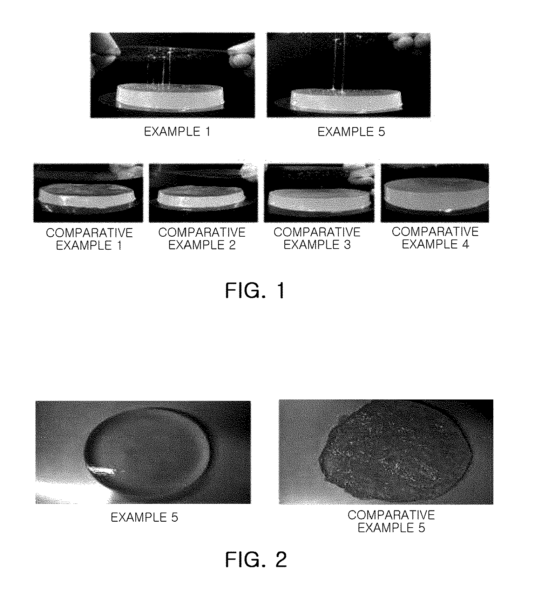

FIG. 1 shows evaluation results of comparing external appearances of mucilage of sticky hydrogels of Examples 1 and 5 prepared according to embodiments of the present disclosure with the naked eye, and hydrogels of Comparative Examples 1 to 4 prepared using polyethylene glycol (PEG) (Comparative Example 1), polyquaternium-7 (Comparative Example 2), and sodium polyacrylate (Comparative Example 3) and polyacrylic acid (Comparative Example 4) having an Na substitution degree lower than 50% instead of sodium polyacrylate as a hydrophilic polymer; and
FIG. 2 is evaluation results of comparing the formulation stability (gel formation) of a sticky hydrogel of Example 5 prepared according to an embodiment of the present disclosure with the naked eye and the formulation stability of a hydrogel of Comparative Example 5 preparing by excessively including 1.5% by weight of a hydrophilic polymer based on total weight.

### [Best Mode]

Exemplary embodiments of the present disclosure will hereinafter be described in detail with reference to the accompanying drawings.

A sticky hydrogel cosmetic composition of an embodiment of the present disclosure is characterized by including 0.1% by weight to 1% by weight of sodium polyacrylate having an Na substitution degree of 50% or higher as a hydrophilic polymer based on total weight.

In the embodiment of the present disclosure, since sodium polyacrylate is a good hydrophilic polymer capable of containing water in an amount up to about 800 times the molecular weight thereof, the sodium polyacrylate is an important component in imparting mucilage's external appearance and moisturizing ability to the sticky hydrogel cosmetic composition of the embodiment of the present disclosure. When the Na substitution degree is less than 50%, it is difficult for the sodium polyacrylate to secure a desired degree of pituitousness (sticky form). The sodium polyacrylate may be added in a range of 0.1% by weight to 1% by weight based on the total weight of the composition. When the sodium polyacrylate is added in a range of less than 0.1% by weight, the desired degree of pituitousness may not be realized. When the sodium polyacrylate is added in a range of more than 1% by weight, a viscosity of the sticky hydrogel cosmetic composition may be too high, or the polyacrylate is trapped between a gelling polymer such that the gelation of the sticky hydrogel cosmetic composition is prevented, and the sticky hydrogel cosmetic composition may obtain a porridge-like consistency.

According to an embodiment of the present disclosure, the sticky hydrogel cosmetic composition may further include 1% by weight to 5% by weight of a gelling polymer, 0.1% by weight to 10% by weight of a skin effective component, 10% by weight to 35% by weight of a polyvalent alcohol, and 0.01% by weight to 0.05% by weight of a gelling rate control agent based on total weight in addition to the hydrophilic polymer.

In the sticky hydrogel cosmetic composition of the embodiment of the present disclosure, examples of the gelling polymer may include galactomannan, glucomannan, guar gum, Locust Bean Gum, pluronic, agar, algin, carrageenan, xanthan, zelan, and mixtures of two or more thereof. The gelling polymer may be mixtures of carrageenan, Locust Bean Gum, and agar. The gelling polymer may be added in a range of 1% by weight to 5% by weight based on the total weight of the composition.

Further, any material may be used as the skin effective component if the material is a whitening component, an anti-wrinkle functional component, an antioxidant component, a moisturizing component, an antibacterial component, or the like, that may increase the absorption of a functional component capable of effectively functioning in skin while improving affinity with skin. Examples of the skin effective component may include: retinol, retinyl palmitate, retinyl acetate, retinoic acid, coenzyme Q1O, elastin, collagen, hyaluronic acid, ceramides, collagen, caffeine, chitosan, ascorbic acid, ascorbyl glucoside, alpha bisabolol, tocopherols, tocopherol acetate, arbutin, niacinamide, adenosine, retinolacetate, vitamins A, D and E, and other natural extracts; natural fermented materials such as the fermented soybean material Natto, etc; and mixtures two or more thereof. However, the skin effective component is not limited thereto. Further, examples of the natural extracts may include one or more selected from the group consisting of aloe, green tea, ginseng, red ginseng, pyroligneous liquor, pine needles, ginkgo leaves, propolis, mulberry leaves, silkworm, Dioscorea batatas, snail secretion filtrate, kakadu plums, camu camu, assai palm, squalane, caviar, broccoli, blueberry, witch hazel, acerola, chlorella, mangosteen, guavas, corni, carrots, caffeine, hamamelis, spirulina, salmon roe, Ecklonia cava Kjellman in Kjellman & Petersen, giant kelp, kelp, portulaca, green laver, agar seaweed, mulberry root, Raspberry, wild berry, fusiformis, sea tangle, edelweiss, chamomile, lavender, peppermint, eucalyptus, lemon balm, oregano, tea tree, scutellaria, Huttuynia cordata, sea buckthorn, and citron.

The skin effective component may be added in a range of 0.1% by weight to 10% by weight based on the total weight of the composition. When the skin effective component is added in a range of less than 0.1% by weight, the skin affinity may be reduced to result in a weak skin-improving effect. When the skin effective component is added in a range of more than 10% by weight, the composition may stimulate skin, or formulation stability of the composition may be reduced to cause a problem of disodorization or discoloration.

Further, if there are materials playing a role of dispersing a gelling polymer as well as a role of moisturization, any of the materials may be used as the polyvalent alcohol. Examples of the polyvalent alcohol include one or more selected from the group consisting of glycerin, ethylene glycol, 1,3-butyleneglycol, propylene glycol, dipropylene glycol, sorbitol, and xylitol. The examples of the polyvalent alcohol may include glycerin. The polyvalent alcohol may be included in a range of 10% by weight to 35% by weight based on the total weight of the composition. When the polyvalent alcohol is included in a range of less than 10% by weight, there may be a problem in that it may be difficult to exhibit a sufficient dispersion effect of the gelling polymer, or water may be volatilized when the composition adheres to skin. When the polyvalent alcohol is included in a range of more than 35% by weight, stickiness of the composition may become excessive, or unit costs of raw materials for the composition may increase.

Examples of the gelling rate control agent may include EDTA, EDTA-2 sodium, potassium citrate, sodium citrate, organic acid salts thereof, organic bases thereof, and mixtures thereof.

In an embodiment of the present disclosure, the sticky hydrogel cosmetic composition may further include an oil and a surfactant as necessary in addition to the above-mentioned components. The oil may be used in a range of 0.1% by weight to 10% by weight based on the total weight of the composition, and the surfactant may be used in a range of 0.1% by weight to 10% by weight based on the total weight of the composition.

Examples of the oil may include one or more selected from the group consisting of: ester-based oils including isopropyl myristate, ethyl laurate, ethyl myristate, isopropyl palmitate, butyl laurate, hexyl laurate, caprylic/capric triglyceride, butylene glycol dicaprylate/dicaprate, isopropyl myristate, triethyl hexanoin, cetyl ethylhexanoate, octyldodecanol, and polyol ester; hydrocarbon-based oils including cyclododecane, isooctane, liquid paraffin, Vaseline, hydrogenated polydecene, cyclooctane, and squalene; waxes including microcrystalline wax, beeswax, lanolin wax, ozokerite wax, candelilla wax, carnauba wax, and synthetic wax; natural oils including jojoba oil, avocado oil, almond oil, olive oil, sesame oil, wheat germ oil, safflower oil, camellia oil, caster oil, grape seed oil, green tea seed oil, macadamia nut oil, palm oil, rose hip oil, hydrogenated oil, argan oil, sea buckthorn oil, argan tree kernel oil, and lavender oil; and silicone oils including cyclomethicone, phenyl trimethicone, cyclopentasiloxane, dimethicone. However, the oil is not limited thereto. The examples of the oil may include olive oil, dimethicone, caprylic/capric triglyceride, cetyl ethylhexanoate, liquid paraffin, and mixtures thereof.

Further, examples of the surfactant may include one or more selected from the group consisting of: polyvalent alcohols including glyceryl monostearate, polyglyceryl-4 isostearate, sorbitan monostearate, and sugar ester monostearate; polyoxyethylene methylpolysiloxane copolymers including ester based PEG-8 dimethicone, PEG-10 dimethicone, PEG-9 methyletherdimethicone, PEG-3 dimethicone, and PEG-11 methyletherdimethicone of fatty acids; poly(oxyethylene· oxypropylene)methylpolysiloxane copolymers including PEG/PPG-20/20 butyletherdimethicone and PEG/PPG-20/20 dimethicone; and alkyl chain·silicone chain branched-type poly(oxyethylene oxypropylene)methylpolysiloxane copolymers including cetyl PEG/PPG-10/1-dimethicone and lauryl PEG-9 polydimethylsiloxyethyl dimethicone. However, the surfactant is not limited thereto.

Further, the sticky hydrogel cosmetic composition of the embodiment of the present disclosure may additionally include other additives such as a preservation agent, a fragrance, a pH control agent and a thickener as necessary.

Examples of the preservation agent may include one or more selected from the group consisting of caprylyl glycol, paraoxybenzoate alkyl ester, benzoic acid, sodium benzoate, sorbic acid, potassium sorbate, phenoxyethanol, parabens, pentanediol, benzoic acid, salicylic acid, dehydroacetic acid and its salts, ethylhexylglycerin, 1,2-hexanediol, Caprylylglycoltropolone, p-oxybenzoate ester, 3-trifluoromethyl-4,4'-dichlorocarbanilide, and 2,4,4'-trichloro-2'-hydroxydiphenyl ether. However, the preservation agent is not limited thereto.

Although examples of the solvent that are usable as a base component of the cosmetic composition of the embodiment of the present disclosure are not particularly limited, the examples of the solvent may include purified water and others, and the cosmetic composition of the embodiment of the present disclosure may include a balance of the solvent.

A sticky hydrogel cosmetic composition according to an embodiment of the present disclosure may be prepared by a preparation process including:
1) mixing a skin effective component, a polyvalent alcohol, a gelling rate control agent, a thickener and a solvent at a temperature of 60°C to 80°C;
2) adding a hydrophilic polymer, a gelling polymer and other additives to the mixture to form a gelatinous solid;
3) coating the gelatinous solid on a support; and
4) shaping the gelatinous solid in an adhesion portion between the gelatinous solid and the support.

The support may be prepared from polyethylene terephthalate, polybutylene terephthalate, polyethylene naphthalate, polyethylene, polypropylene, polybutadiene, ethylene vinyl acetate copolymers, polyvinyl chloride, or mixtures thereof. After the gelatinous solid is coated and formed a coated layer on the support, a coating face of the gelatinous solid is coated with a separable nonwoven fabric, paper, or film to prevent water from evaporating from the gelatinous solid. The coated layer may preferably have a thickness of 0.05 cm to 0.3 cm.

### [Mode for Invention]

Hereinafter, exemplary embodiments of the present disclosure are described more in detail through the following Examples and Comparative Examples. However, such embodiments are provided for illustrative purposes only, and the scope of the present invention should not be limited thereto in any manner. Further, it should be understood that the present disclosure is not limited to the above descriptions since other various modifications of the present disclosure may occur to persons having ordinary knowledge in the related art of the present disclosure.

### Examples 1 to 7

Sticky hydrogels of Examples 1 to 7 were prepared using components suggested in Tables 1 and 2.

First, as shown in Tables 1 and 2, polyvalent alcohols, skin effective components, a gelling rate control agent, a thickener, and a solvent were mixed together at a temperature of 60°C to 80°C to, and then gelling polymers, a hydrophilic polymer, and other additive components were added to the mixture to obtain a hydrogel mixture. The hydrogel mixture was coated on a film, and the hydrogel mixture coated on the film was formed to have a desired shape without an aging process. In this manner, sticky hydrogels of Examples 1 to 7 were prepared. Commercially available NP700 (having an Na substitution degree of 50%, produced by SHOWA DENKO) was used as sodium polyacrylate for Examples 1 to 4, and commercially available NP600 (having an Na substitution degree of 65%, produced by SHOWA DENKO) was used as sodium polyacrylate for Examples 5 to 7.

**[Table 1]**

| No. | Component Types | Component Name (wt. %) | Example 1 | Example 2 | Example 3 | Example 4 | Example 5 |
|---|---|---|---|---|---|---|---|
| 1 | Gelling polymer | Carrageenan | 0.5 | 0.5 | 1.0 | 2.0 | 2.5 |
| | | Agar | 0.2 | 0.5 | 0.5 | 1.5 | 0.3 |
| | | Locust Bean Gum | 0.3 | 0.5 | 0.5 | 1.5 | 0.5 |
| 2 | Polyvalent alcohols | Glycerin | 20 | 15 | 5 | 25 | 20 |
| | | Sorbitol | - | - | 10 | 5 | 2 |
| 3 | Skin effective components | Snail secretion filtrate | 5 | 2 | 3 | 4 | 5 |
| | | Adenosine | 0.02 | 0.02 | 0.04 | 0.04 | 0.04 |
| | | Hydrolyzed collagen | 0.1 | 0.1 | - | 0.1 | - |
| | | Portulaca extract | 0.1 | 0.1 | - | 0.1 | - |
| 4 | Hydrophilic polymer | Sodium polyacrylate | 0.2 | 0.3 | 0.3 | 0.5 | 0.5 |
| 5 | Gelling rate control agent | Sodium EDTA | 0.01 | 0.02 | 0.02 | 0.01 | 0.01 |
| 6 | Thickener | Cellulose | 0.1 | 0.1 | 0.1 | 0.1 | 0.1 |
| 7 | Surfactant | Polysorbate 60 | 0.15 | 0.15 | 0.15 | 0.15 | 0.15 |
| 8 | pH control agent | Citric acid | 0.15 | 0.15 | 0.15 | 0.15 | 0.15 |
| 9 | Fragrance | Fragrance | 0.01 | 0.01 | 0.01 | 0.01 | 0.01 |
| 10 | Solvent | Purified water | 73.16 | 80.55 | 79.23 | 59.84 | 68.74 |

**[Table 2]**

| No. | Component types | Component name (wt.%) | Example 6 | Example 7 |
|---|---|---|---|---|
| 1 | Gelling polymer | carrageenan | 2.5 | 2.5 |
| | | Agar | 0.3 | 0.3 |
| | | Locust Bean Gum | 0.5 | 0.5 |
| 2 | Polyvalent alcohols | Glycerin | 20 | 20 |
| | | Sorbitol | 2 | 2 |
| 3 | Skin effective components | Snail secretion filtrate | 5 | 5 |
| | | Adenosine | 0.04 | 0.04 |
| 4 | Hydrophilic polymer | Sodium polyacrylate | 0.5 | 0.5 |
| 5 | Gelling rate control agent | Sodium EDTA | 0.01 | 0.01 |
| 6 | Thickener | Cellulose | 0.1 | 0.1 |
| 7 | Surfactant | Polysorbate 60 | 0.15 | 0.15 |
| 8 | pH control agent | Citric acid | 0.15 | 0.15 |
| 9 | Fragrance | Fragrance | 0.01 | 0.01 |
| 10 | Oil | caprylic/capric triglyceride | 5 | 10 |
| 10 | Solvent | Purified water | 63.74 | 58.74 |

### Comparative Example 1

A hydrogel of Comparative Example 1 was prepared by the same method as Example 1 except that polyethylene glycol (PEG) was used instead of sodium polyacrylate.

### Comparative Example 2

A hydrogel of Comparative Example 2 was prepared by the same method as Example 1 except that polyquaternium-7 (quaternary ammonium salts polymerized by acrylamide and diallyl dimethyl ammonium chloride monomers) was used instead of sodium polyacrylate.

### Comparative Example 3

A hydrogel of Comparative Example 3 was prepared by the same method as in Example 1 except that NP800 having an Na substitution degree of 35%, produced by SHOWA DENKO, was used as sodium polyacrylate.

### Comparative Example 4

A hydrogel of Comparative Example 4 was prepared by the same method as in Example 1 except that polyacrylic acid Jurymer AC-10H in which Na was not substituted and which was produced by Nihon Junyaku Co., Ltd was used instead of sodium polyacrylate.

### Comparative Example 5

A hydrogel of Comparative Example 5 was prepared by the same method as in Example 1 except that 1.5% by weight of sodium polyacrylate and 71.86% by weight of purified water were added.

### Test Example 1: Evaluating external appearances of mucilages

In order to evaluate external appearances of mucilages of the prepared hydrogels of Examples 1 to 7 and Comparative Examples 1 to 5, viscosity extents of the mucilages were evaluated with the naked eye while detaching PET films from the hydrogels after attaching the PET films to upper portions of the hydrogels. The evaluation results were illustrated in FIG. 1.

Resultingly, the hydrogels of Examples 1 to 7 and Comparative Example 5 prepared using sodium polyacrylate having an Na substitution degree of 50% or higher had the viscosity of sticky mucilage as shown in the results of Examples 1 and 5 shown in FIG 1. However, such viscous properties were not shown in the hydrogels of Comparative Examples 1 to 4 prepared by using polyethylene glycol (PEG), polyquaternium-7, or sodium polyacrylate having an Na substitution degree of less than 50% as a hydrophilic polymer.

### Test Example 2: Testing formulation stability

Whether the prepared hydrogels of Examples 1 to 7 and Comparative Examples 1 to 5 were stable in formulation (in forming the gels) or not was evaluated using the naked eye. The evaluation results were shown in FIG. 2.

Resultingly, as shown in the results of Example 5 in FIG. 2, formulation stability was good in the hydrogels of Examples 1 to 7 and Comparative Examples 1 to 4 prepared in such a manner that the hydrogels were contained in amounts of 0.2% by weight to 0.5% by weight of a hydrophilic polymer component. However, gelation was not observed in the hydrogel of Comparative Example 5 containing a hydrophilic polymer component in an excessive amount of 1.5% by weight, but the hydrogel of Comparative Example 5 obtained a porridge-like consistency.

As described above, the hydrogel cosmetic composition of the present disclosure which includes 0.1% by weight to 1% by weight of sodium polyacrylate having an Na substitution degree of 50% or higher as a hydrophilic polymer may form a hydrogel having high formulation stability and sticky mucilage. Therefore, when the sticky hydrogel cosmetic composition is applied to skin, the sticky hydrogel cosmetic composition may easily adhere to the skin and allow the skin to maximally adsorb moisture and components of the sticky hydrogel cosmetic composition. Thus, the sticky hydrogel cosmetic composition may be widely utilized in the development of various cosmetics such as mask packs.

While exemplary embodiments have been shown and described above, it will be apparent to those skilled in the art that modifications and variations could be made without departing from the scope of the present invention as defined by the appended claims. The exemplary embodiments should be considered in descriptive sense only and not for purposes of limitation. Therefore, the scope of the invention is defined not by the detailed description but by the appended claims, and all differences within the scope will be construed as being included in the present invention.

## Claims

1. A sticky hydrogel cosmetic composition comprising 0.1% by weight to 1% by weight of sodium polyacrylate having an Na substitution degree of 50% or higher as a hydrophilic polymer based on total weight.

2. The sticky hydrogel cosmetic composition of claim 1, further comprising 1% by weight to 5% by weight of a gelling polymer, 0.1% by weight to 10% by weight of a skin effective component, 10% by weight to 35% by weight of a polyvalent alcohol and 0.01% by weight to 0.05% by weight of a gelling rate control agent based on total weight.

3. The sticky hydrogel cosmetic composition of claim 1, further comprising 0.1% by weight to 10% by weight of an oil, 0.1% by weight to 10% by weight of a surfactant, or both thereof based on total weight.

4. The sticky hydrogel cosmetic composition of claim 1, further comprising at least one additive selected from the group consisting of a preservation agent, a fragrance, a pH control agent, and a thickener.
